# EUROPÄISCHE PATENTSCHRIFT

(11) **EP 1 084 569 B1**
(45) Veröffentlichungstag und Bekanntmachung des Hinweises auf die Patenterteilung: **05.03.2003**
(21) Anmeldenummer: 99938132.0
(22) Anmeldetag: 07.06.1999
(51) Int. Cl.: H04N 5/00, A61P 5/26

(54) **ANDROGENE IN KOMBINATION MIT GESTAGENE ZUM AUSGLEICH EINES TESTOSTERON-DEFIZITS MIT SCHUTZ DER PROSTATA**
ANDROGENS IN COMBINATION WITH GESTAGENS FOR COMPENSATING FOR A TESTOSTERONE DEFICIENCY IN MEN WHILE SIMULTANEOUSLY PROTECTING THE PROSTATE
ANDROGENES EN COMBINAISON AVEC GESTAGENES POUR COMPENSER UN DEFICIT EN TESTOSTERONE CHEZ L'HOMME ET OFFRANT SIMULTANEMENT UNE PROTECTION DE LA PROSTATE

(30) Priorität: 09.06.1998 DE 19825591
(43) Veröffentlichungstag der Anmeldung: 21.03.2001
(73) Patentinhaber: Jenapharm GmbH & Co. KG, 07745 Jena (DE)
(72) Erfinder: HÜBLER, Doris, D-07407 Schmieden (DE); OETTEL, Michael, D-07743 Jena (DE); SOBEK, Lothar, D-07743 Jena (DE); ELGER, Walter, D-14195 Berlin (DE); AL-MUDHAFFAR, Abdul-Abbas, D-07747 Jena (DE)
(74) Vertreter: Leybach, Holger Hugo
(86) Internationale Anmeldenummer: DE9901652
(87) Internationale Veröffentlichungsnummer: WO99065228

(56) Entgegenhaltungen:
- EP-A- 0 747 054
- WO-A-91/00095
- WO-A-91/00731
- WO-A-94/27610
- WO-A-99/46279
- US-A- 4 310 523
- US-A- 4 598 072
- OKADA K ET AL: "Study of the effect of an anti-androgen (oxendolone) on experimentally induced canine prostatic hyperplasia. I. Morphological analysis." UROLOGICAL RESEARCH, (1988) 16 (2) 67-72., XP000874665
- HABENICHT U F ET AL: "Selective inhibition of androstenedione -induced prostate growth in intact beagle dogs by a combined treatment with the antiandrogen cyproterone acetate and the aromatase inhibitor 1-methyl-androsta-1,4-diene-3,17-dione (1-methyl-ADD)." PROSTATE, (1989) 14 (4) 309-22., XP000874661
- HABENICHT U F ET AL: "Synergic inhibitory effects of the aromatase inhibitor 1-methyl-androsta-1, 4-diene-3, 17-dione and the antiandrogen cyproterone acetate on androstenedione -induced hyperplastic effects in the prostates of castrated dogs." PROSTATE, (1987) 11 (2) 133-43., XP000874660
- MURAKOSHI M. ET AL: "Inhibition of steroid-induced prostatic hyperplasia in rats by treatment with anti-androgen (TZP-4238)." ENDOCRINE JOURNAL, (1993) 40/4 (479-488)., XP000874644
- TAMMELA T.: "Benign prostatic hyperplasia. Practical treatment guidelines." DRUGS AND AGING, (1997) 10/5 (349-366)., XP000874648

## Beschreibung

Die Erfindung betrifft die Herstellung eines Kombinationspräparates zum Ausgleich eines absoluten und relativen Testosteron-Defizits beim Mann mit gleichzeitiger Prophylaxe für die Entstehung einer beningnen Prostatahyperplasie (BPH) bzw. des Prostata-Karzinoms, enthaltend ein natürliches oder synthetisches Androgen in Kombination mit einem Gestagen.

Verschiedene endokrine Funktionen verändern sich im Verlauf des Alterungsprozesses.
Der normale Alterungsprozeß beim Mann geht einher mit einer Abnahme der Hodenfunktion, insbesondere mit einer Abnahme der Serum-Testosteron-Spiegel.
Die Serum-Testeron-Sekretion ist für die sekundären Geschlechtsmerkmale, Libido und Potenz verantwortlich und hat auch einen Einfluß auf die Stimmungslage und intellektuelle Fähigkeiten, auf die Erythropoese, den Knochenstoffwechsel, den Proteinanabolismus und die Muskelmasse, die Fettverteilung sowie bestimmte ZNS-Funktionen.
Bei Absinken des Serum-Testosteron-Spiegels können klinisch eine Abnahme der Libido und der Potenz sowie Müdigkeit, Verminderung der Muskelmasse, Osteoporose, Hitzewallungen, Schweißausbrüche und leichte Anämie auftreten.
Allerdings werden Androgenen für die Entstehung und Manifestation sowohl der benignen Prostatahyperplasie (BPH) als auch des Prostatakarzinoms eine wesentliche Rolle zugeschrieben.
Im höheren Lebensalter treten Erkrankungen der Prostata gehäuft auf. Bei 50 % der Männer über 50 Jahre kommt es zu einem gutartigen Wachstum der Prostata (BPH).
Hypogonadale Männer oder Kastraten entwickeln nie eine BPH.
Geller, J.: Androgen inhibition and BPH. In: Bhasin et al. (Hrsg.):Pharmacology, biology, and clinical applications of androgens John Wiley, New York (1996).

Allerdings bestehen bei Männern mit und ohne BPH keine Unterschiede der Androgenkonzentrationen im Serum [Lee, C., Prostate 6 Supple, 52-56 (1996); Levine, A.C. Trends Endocrinol. Metab. 6, 128-132 (1995); Serio, M. and Fiorelli, G. Mol.Cell.Endocrinol.78,, C77-C81 (1991), Cunningham, G.R.: Overview of androgens on the normal and abnormal prostate. In: Bhasin et al. (Hrsg.): Pharmacology, biology, and clinical applications of androgens. John Wiley, New York (1996)], so daß offensichtlich der zelluläre Metabolismus von Testosteron zu 5α-Dihydrotestosteron (DHT) und Estradiol in der Prostata zusammen mit lokalen Wachstumsfaktoren für die Entstehung sowohl der benignen Prostatahyperplasie (BPH) als auch des Prostatakarzinoms von entscheidender Bedeutung ist.
Nachweislich treten sowohl bei Männern über 50 als auch bei jüngeren Männern mit verschiedenen chronischen Krankheiten und Dauerstreß alle aufgezeigten klinischen Symptome schon gehäuft bei Serum-Testosteron-Spiegeln an der unteren Normgrenze von 12,0 bis 15 nmol/l auf.

Aus der Literatur bzw. Patentliteratur ist bekannt, androgen-abhängige systemische Erkrankungen, wie beispielsweise die BHP und das Prostatakarzinom mit Antiandrogenen alleine - W94/26767 A1- oder in Kombination mit Testolacton als Aromatase-Hemmer - DE 3121152 A1 -, mit Testosteron-5α-Reduktase-Hemmer alleine - EP 0 547 691 A1; WO 95/13077 A1 - oder in Kombination mit Antiestrogenen und/oder Aromatase-Hemmern WO 91/00731 A1 zu behandeln.
Eine Testosteron-Replacement-Therapie ohne Gefahr für die Prostata ist nicht aufgezeigt.

In der Fachliteratur wird aufgezeigt daß eine Androgensubstitution auch im Alter das physische und psychische Wohlbefinden verbessert [Bagatell et al. J. Clin. Endocrin. Metab. 79: 561 - 567 (1994); Tenover, J.S. Endocrinology and Metabolism Clinics of North America 23: 878 - 892 (1994)].
Jedoch wird die Androgensubstitution bei älteren Männern mit erniedrigten Serum-Testosteron-Spiegeln der unterschiedlichsten Ursachen gegenwärtig noch kontrovers diskutiert und immer das erhöhte Risiko von Erkrankungen der Prostata durch Überstimulierung in den Vordergrund gestellt.
Von einer Androgen - Replacement-Therapie des älteren oder frühzeitig gealterten Mannes in Analogie zur postmenopausalen Hormonsubstitution der Frau wird daher gewarnt [Rolf, C. und E. Nieschlag: Seneszenz In E. Nieschlag und H.M. Behre (Hrsg.): Andrologie - Grundlagen und Klinik der reproduktiven Gesundheit des Mannes.Springer 1996; Jackson, J.A. et al. Arch. Intern. Med. 149: 2365 - 2366(1989); Jockenhövel, F. Androgensubstitution des älteren Mannes. In: Allolio und Schulte (Hrsg): Praktische Endokrinologie. Urban & Schwarzenberg, München S. 416 -419 (1996].
Auch Holmäng,S. et al. Prostate 23, 99-106 (1996) konnten z.B. nach 8-monatiger Therapie von 23 Männern im Alter von 40 - 65 Jahren mit Testosteronundecanoat (160 mg / Tag) eine Größenzunahme der Prostata um 12 % feststellen.
In Studien zur männlichen Kontrazeption mit Testosteronenanthat wurde an jungen Männern unter exogener Testosteronapplikation mittels transrektaler Ultraschalluntersuchungen eine Vergrößerung der Prostata gefunden [Wu, C. W. et al. Fertility and Sterility 65, 626 - 636 (1996); Wallace, E.M. et al. Int. J. Androl. 16: 35 - 40 (1993)].

Die Patentschrift DE 196 10 645 A1 beschreibt die Verwendung von Dehydroepiandrosteron in Kombination mit Aromatsehemmern zur Behandlung eines relativen und absoluten Andogenmangels beim Mann (Hypoandrogenismus). Aromatasehemmer im Sinne dieser Patentschrift sind alle diejenigen Verbindungen, die die Bildung von Estrogenen aus deren metabolischen Vorstufen ( hier DHEA) durch Hemmung des Enzyms Aromatase (Hemmung der Biosynthese) verhindern.
Eine Androgentherapie mit gleichzeitigem Schutz der Prostata ist jedoch nicht aufgezeigt.

Die Patentschrift WO 97/ 29735 beansprucht Androgene, Antiandrogene, Estrogene oder Antiestrogene enthaltende transdermale Systeme, einzeln oder in Kombination, zur Androgentherapie bei Defizit des Testosteron-Spiegels beim hypogonadalen Mann, zur Hormonsubstitutionstherapie bei postmenopausalen Frauen und zur hormonelfen Kontrazeption beim Mann und bei der Frau.
Auch hier ist eine Androgentherapie mit gleichzeitigem Schutz der Prostata nicht ableitbar.

Aufgabe der vorliegenden Erfindung ist es , geeignete Mittel zum Ausgleich eines absoluten und relativen Testosteron-Defizits beim Mann mit gleichzeitigem Schutz der Prostata bereitzustellen und dabei die oben genannten Nachteile und Wirkungen zu vermeiden.

Die Aufgabe wird durch die Verwendung nach Anspruch 1 zum Ausgleich eines absoluten und relativen Testosteron-Defizits mit gleichzeitiger Therapie der benignen Prostatahyperplasie (BPH) gelöst.

Die erfindungsgemäß verwendeten Kombinationen sind vorzugsweise dadurch gekennzeichnet, daß das natürliche Androgen eine der Substanzen Testosteron, Testosteronundecanoat, Dehydroepiandrosteron, Dehydroepiandrosteron-Sulfat, Testosteronpropionat, Testosteronenanthat, Testosteronbuciclat, Testosteroncypionat oder Androstendion
und das synthetische Androgen eine der Substanzen
17-Methyltestosteron, Fluoxymesteron, Danazol, Mesterolon, Nandrolondecanoat, Nandrolonphenylpropionat, Oxandrolon, Oxymetholon, Stanazolol ist.
Es erweist sich als vorteilhaft, daß die Dosierung des Androgens , beispielsweise von Testosteronundecanoat 250 bis 1500 mg i.m. alle 4 bis 14 Wochen beträgt.
Besonders vorteilhaft ist dabei die Gabe von 1000 mg alle 9 bis 10 Wochen an Testosteronundecanoat.

Die erfindungsgemäß verwendeten Kombinationen sind vorzugsweise dadurch gekennzeichnet, daß die Gestagenkomponente eine der Substanzen Dienogest, Levonorgestrel, Gestoden, Desogestrel, Norgestimat, Norethisteron, Norethisteronacetat, Levonorgestrel oder.

Progesteron, Chlormadinonacetat, Cyproteronacetat, Medroxyprogesteronacetat, Megestrolacetat, Dydrogesteron, Trimegeston oder Nomegestrol ist.

Vorteilhaft ist dabei, daß die Dosierung des Gestagens 20 µg bis 20 mg beträgt.

Die erfindungsgemäß hergestellten Kombinationspräparaten können in unterschiedlichen Zubereitungs- bzw. Applikationsformen vorliegen.

Die pharmazeutischen Zubereitungsformen können die Kombinationen als einheitliche Form darstellen oder auch zwei getrennte Formulierungen beinhalten. Dabei können es sich um Peroralia, z.B. Tabletten, Kapseln und Dragees, um perkutane Zubereitungsformen, z.B. Transdermale Therapeutische Systeme (TTS) oder Gele, Sprays oder Salben, um intranasale Zubereitungsformen wie Nasenspray oder Nasentropfen, rektale Zubereitungsformen wie Suppositorien und um Parenteralia, z.B.: Implantate, Presslinge und Ampullen handeln.

Die Zubereitungsformen werden in an sich bekannter Weise unter Verwendung üblicher Hilfs- und Trägerstoffe hergestellt, wie beispielsweise im "Remington's Pharmaceutical Sciences Handbook, Hack Pub. co., N. Y., USA" beschrieben sind.

Es wurde eine pharmazeutische Kombinationen zum Ausgleich eines absoluten und relativen Testosteron-Defizits beim Mann mit gleichzeitiger Prophylaxe der benignen Prostatahyperplasie (BPH) gefunden.
Im Vergleich zur erfindungsgemäßen Kombination kann jeder Wirkstoff alleine das gewünschte Ziel nicht in diesem Maße und nur unter signifikanten Nebenwirkungen erreichen.

Mit den erfindungsgemäßen Kombinationen wird die durch die Gabe der Androgene hervorgerufene DHT-Stimulierung bzw. Überstimulierung in der Prostata durch die aufgezeigten Komponenten, wie Gestagene, Antigestagene, Antiestrogene, GnRH-Analoga, Testosteron-5α-Reduktase-Hemmer, α-Andreno-Rezeptorblocker oder Phosphodiestrase-Hemmer ausgeglichen.

Es wurde am Beispiel der Hemmung der Androgen-abhängigen Zellproliferation in LNCaP Prostatazellen der biologische Wirkmechanismus der Kombination 17β-Hydroxy-17α-methyl-estra-4,9,11-trien-3-on
(R 1881) + das 17α-Cyano- methyl-17β-hydroxy-estra-4,9-dien-3-on (DIENOGEST = DNG) geprüft.

Hierzu wurde die menschliche Prostatakarzinomzelle LNCaP unter Routinebedingungen in Dulbecco's modifiziertem Eagle medium (DMEM) unter Zusatz von 10% FCS (Fetal calf serum) kultiviert.
Die Zellen wurden dann 2 bis 6 Passagen mit DMEM und 10 % DCC-FCS (steroid depleted FCS) gezüchtet, bevor sie im Wachstumsassay mit 5 % DCC-FCS eingesetzt wurden.
Für den Versuch wurden die Zellen in 24-weil Platten ausgesät (10 000 Zellen/well und ml).
Nach 24 Stunden wurden die in Ethanol gelösten Steroide in frischem Versuchsmedium (Endkonzentration an Ethanol 0,1 %) dazugegeben und die Zellen wurden 7 Tage bei 37° (5 % CO₂) inkubiert. Die Zellzahl wurde nach dieser Zeit in einem Zellzähl- und Analysensystem (CASY, Schärfe System GmbH) bestimmt.

Aus der Abbildung 1 ist ersichtlich, daß das sehr starke Androgen 17β-Hydroxy-17α-methyl-estra-4,9,11-trien-3-on (R1881) ein Zellwachstum induziert. In Abhängigkeit von der Dosis der zweiten Komponente - hier das Gestagen DNG - wird jedoch die Androgen-abhängige Zellproliferation der Prostata-Karzinom-Zellinie gehemmt. Die Dienogest-Wirkung ist vornehmlich peripher an den Geschlechtsorgane (Oettel, M. et a.: Der Einfluß einer Ethinylestradiol-Dienogest-Kombination auf die Serum-Androgen-Konzentrationen, Zentralblatt Gynäkol 119, 597-606, 1997).

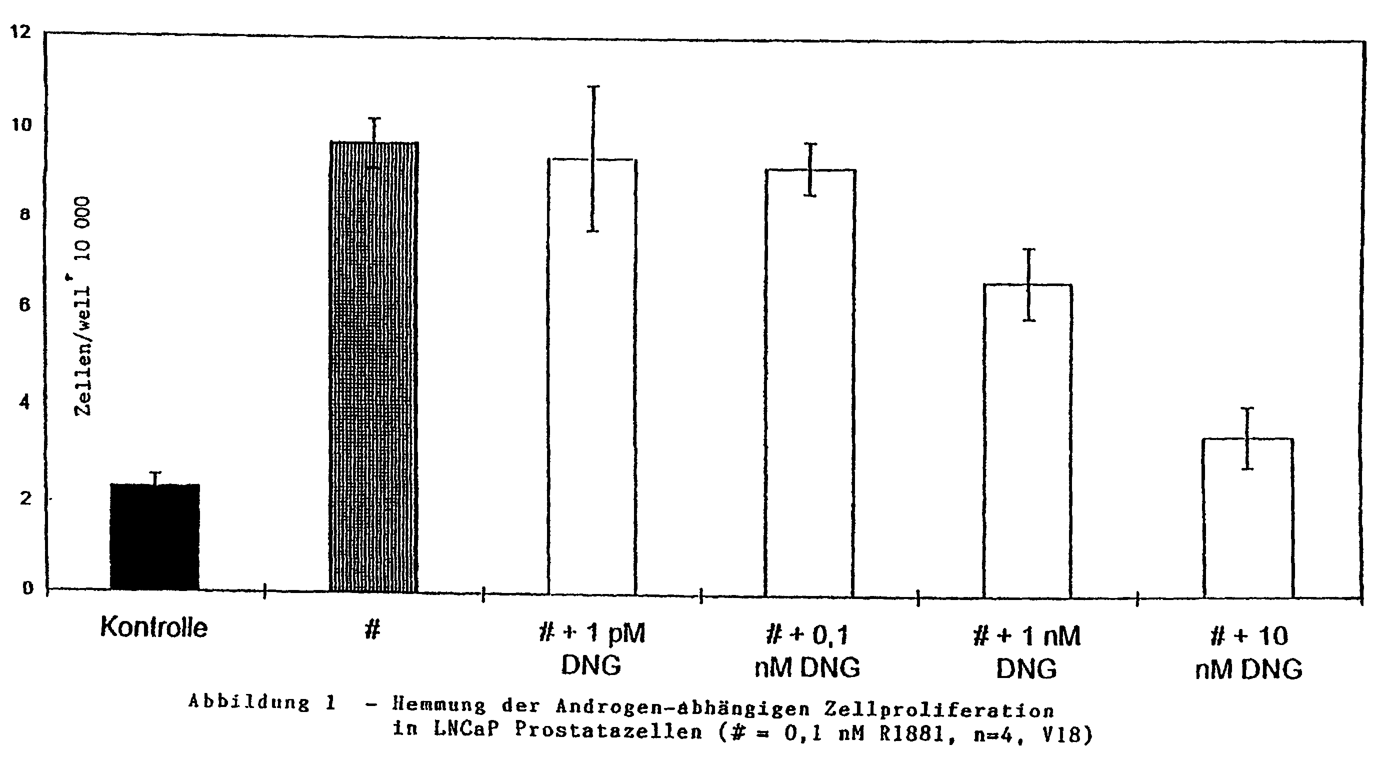

Ferner wurde die Hemmung eines durch Androgene induzierten Prostatawachstums, welches mit einer erfindungsgemäßen Kombination behandelt wurde, im Tierexperiment untersucht.

Hierzu wurden 5 männliche NMRI Mäuse von M⌀llegaard Breeding Centre Deutschland GmbH, Schönwalde im Gewicht von 28-30 g kastriert.
Zwei Wochen nach der Kastration wurde den Kontrolltieren Testosteronpropionat (TP) allein - 0,1 mg/Tier verabreicht.
Ebenfalls zwei Wochen nach Kastration wurden die Versuchstiere eine Woche täglich mit Testosteronpropionat (TP) 0,1 mg/Tier/Tag s.c. und gleichzeitig mit folgenden Gestagenen und folgenden Dosierungen oral behandelt:
Cyproteronacetat (CPA) in den Dosierungen von 0,1; 0,3; 1; 3 mg/Tier/Tag
Dienogest (DNG) in den Dosierungen von 0,3; 1; 3; 10 mg/Tier/Tag,
Chlormadinonacetat (CMA) in den Dosierungen 0,3; 1; 3; 10 mg/Tier/Tag.
Am Versuchsende wurden die Prostatagewichte der Mäuse ermittelt und die Versuchsgruppen verglichen.

In Tabelle 1 sind die ermittelten Prostatagewichte der behandelten Mäuse während des Versuchsverlaufes aufgezeigt.

| **Prostatagewichte von kastrierten Mäusen in mg (Mittelwert ± S.D.) nach kombinierter Testosteron/Gestagen - Behandlung (n = 5 Tiere / Gruppe)** | | | | | | |
|---|---|---|---|---|---|---|
| **Gestagen-Dosierung ./. Gruppierungen** | | **0,1 (mg/Tier/ Tag)** | **0,3 (mg/Tier/ Tag)** | **1,0 (mg/Tier/T ag)** | **3,0 (mg/Tier/ Tag)** | **10,0 (mg/Tier/ Tag)** |
| **intakte Kontrolle** | 3,0±0,8 | | | | | |
| **kastrierte Kontrolle** | 1,7±0,7 | | | | | |
| **TP Kontrolle** | 4,4±0,7 | | | | | |
| **TP + CPA** | | 2,7±1,2* | 3,3±1,3 | 2,2±1,1* | 2,3±0,8* | 2,7±0,7* |
| **TP + DNG** | | | 2,7±0,7* | 3,4±0,5* | 3,1±0,7* | 2,4 ±0,9* |
| **TP + CMA** | | | 3,0±1,0* | 3,3±0,8 | 3,0±1,1* | 2,5±0,4* |

| | | | | | | |
|---|---|---|---|---|---|---|
| * signifikant p ≥ 0.05 (Substanzgruppe vs. TP) | | | | | | |

Aus Tabelle 1 ist ersichtlich, daß die gewählte reine Testosterondosis - 0,1 mg/Tier/Tag eine deutliche Zunahme des Prostatagewichtes gegenüber den kastrierten und den intakten Kontrolltieren verursacht. Die Kombination Androgen/ Gestagen vermindert die Androgen-bedingte Zunahme des Prostatagewichtes - abhängig von der Dosierung des Gestagens - bis in den Bereich der Prostatagewichte von intakten Vergleichstieren.

Damit ist nachweislich der Ausgleich des relativen Testosteron-Defizits und der gleichzeitige Schutz der Prostata gewährleistet.

Mit den erfindungsgemäßen Kombinationen werden Arzneimittel zur Verfügung gestellt, welche ein relatives Testosteron-Defizit beim Mann ausgleichen und gleichzeitig die Prostata schützen.

## Patentansprüche

1. Verwendung eines natürlichen oder synthetischen Androgens in Kombination mit einer Komponente aus der Gruppe der Gestagene, zur Herstellung eines Kombinationspräparates zum Ausgleich eines absoluten oder relativen Testosteron-Defizits mit gleichzeitiger Prophylaxe für die Entstehung einer benignen Prostatahyperplasie (BPH) bzw. eines Prostata-Karzinoms.

2. Verwendung nach Anspruch 1,
**dadurch gekennzeichnet, daß**
die natürliche Androgenkomponente Testosteron, Testosteronundecanoat, Dehydroepiandrosteron, Dehydroepiandrosteron-Sulfat, Testosteronpropionat, Testosteronenanthat, Testosteronbuciclat, Testosteroncypionat oder Androstendion ist.

3. Verwendung nach Anspruch 1,
**dadurch gekennzeichnet, daß**
die synthetische Androgenkomponente 17-Methyltestosteron, Fluoxymesteron, Danazol, Mesterolon, Nandrolondecanoat, Nandrolonphenylpropionat, Oxandrolon, Oxymetholon, Stanazolol ist.

4. Verwendung nach Anspruch 1,
**dadurch gekennzeichnet, daß**
die Gestagenkomponente Dienogest, Levonorgestrel, Gestoden, Desogestrel, Norgestimat, Norethisteron, Norethisteronacetat, Levonorgestrel oder Progesteron, Chlormadinonacetat, Cyproteronacetat, Medroxyprogesteronacetat, Megestrolacetat, Dydrogesteron, Trimegeston oder Nomegestrol ist.

5. Verwendung nach
mindestens einem der Ansprüche 1 bis 4,
**dadurch gekennzeichnet**, daβ Kombinationspräparat in Form von Tabletten, Kapseln, Dragees, Transdermalen-Therapie-Systemen, Ampullen, Suppositorien, Gelen, Salben, Nasentropfen, Implantaten, Presslingen oder bioabbaubaren Mikrospheren vorliegt.

## Claims

1. Use of a natural or synthetic androgen in combination with a component from the group consisting of the gestagens, for the production of a combination preparation for the compensation of an absolute or relative testosterone deficit with simultaneous prophylaxis for the development of benign prostate hyperplasia (BPH) or prostate carcinoma.

2. Use according to Claim 1, **characterized in that** the natural androgen component is testosterone, testosterone undecanoate, dehydroepiandrosterone, dehydroepiandrosterone sulphate, testosterone propionate, testosterone enanthate, testosterone buciclate, testosterone cypionate or androstenedione.

3. Use according to Claim 1, **characterized in that** the synthetic androgen component is 17-methyltestosterone, fluoxymesterone, danazole, mesterolone, nandrolone decanoate, nandrolone phenylpropionate, oxandrolone, oxymetholone, stanazolol.

4. Use according to Claim 1, **characterized in that** the gestagen component is dienogest, levonorgestrel, gestodene, desogestrel, norgestimate, norethisterone, norethisterone acetate, levonorgestrel or progesterone, chlormadinone acetate, cyproterone acetate, medroxyprogesterone acetate, megestrol acetate, dydrogesterone, trimegestone or nomegestrol.

5. Use according to at least one of Claims 1 to 4, **characterized in that** the combination preparation is present in the form of tablets, capsules, coated tablets, transdermal therapy systems, ampoules, suppositories, gels, ointments, nasal drops, implants, pellets or biodegradable microspheres.

## Revendications

1. Utilisation d'un androgène naturel ou synthétique en association avec un composant choisi dans le groupe des progestatifs, pour la fabrication d'une préparation comprenant une association de plusieurs substances actives, destinée à compenser une déficience relative ou absolue en testostérone avec prophylaxie simultanée de l'apparition d'une hyperplasie bénigne de la prostate (BPH) ou d'un carcinome de la prostate.

2. Utilisation selon la revendication 1, **caractérisée en ce que** le composant androgène naturel est la testostérone, la testostérone undécanoate, la déhydroépiandrostérone, la déhydroépandrostérone sulfate, la testostérone propionate, la testostérone énanthate, la testostérone buciclate, la testostérone cypionate ou l'androstènedione.

3. Utilisation selon la revendication 1, **caractérisée en ce que** le composant androgène synthétique est la 17-méthyltestostérone, la fluoxymestérone, le danazol, la mestérolone, la nandrolone décanoate, la nandrolone phénylpropionate, l'oxandrolone, l'oxymétholone, le stanazolol.

4. Utilisation selon la revendication 1, **caractérisée en ce que** le composant progestatif est le diénogest, le lévonorgestrel, le gestodène, le désogestrel, le norgestimate, la noréthistérone, la noréthistérone acétate, le lévonorgestrel ou la progestérone, la chlormadinone acétate, la cyprotérone acétate, la médroxyprogestérone acétate, le mégestrol acétate, la dihydrogestérone, la trimégestone ou le nomégestrol.

5. Utilisation selon au moins l'une quelconque des revendications 1 à 4, **caractérisée en ce que** la préparation consistant en une association de plusieurs substances actives se trouve sous forme de comprimés, gélules, dragées, systèmes thérapeutiques transdermiques, ampoules, suppositoires, gels, pommades, gouttes nasales, implants, pellets ou microsphères biodégradables.
